(19) Europäisches Patentamt / European Patent Office / Office européen des brevets

(11) **EP 3 495 491 A1**

(12) **EUROPEAN PATENT APPLICATION**
published in accordance with Art. 153(4) EPC

(43) Date of publication:
**12.06.2019 Bulletin 2019/24**

(51) Int Cl.:
*C12P 21/02* (2006.01)   *C12N 1/00* (2006.01)

(21) Application number: **17836989.8**

(22) Date of filing: **02.08.2017**

(86) International application number:
**PCT/JP2017/027958**

(87) International publication number:
**WO 2018/025886 (08.02.2018 Gazette 2018/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.08.2016 JP 2016151763**

(71) Applicant: **Spiber Inc.**
**Yamagata 997-0052 (JP)**

(72) Inventors:
• **KURACHI Kenji**
**Tsuruoka-shi**
**Yamagata 997-0052 (JP)**
• **KINOSHITA Mihoko**
**Tsuruoka-shi**
**Yamagata 997-0052 (JP)**

(74) Representative: **Handley, Matthew Edward et al**
**Venner Shipley LLP**
**200 Aldersgate**
**London EC1A 4HD (GB)**

(54) **METHOD FOR PRODUCING RECOMBINANT PROTEIN**

(57)    The invention relates to a method for production of a recombinant protein using recombinant cells that express the recombinant protein under the control of an inducible promoter, the method including continuous culturing with addition of fresh culture medium to a portion of the culture solution in which the recombinant cells have been grown, wherein the recombinant cells whose expression of the recombinant protein has been induced are not reused.

## Fig.1

SEQUENCE LISTING

<110>  Spiber Inc.
       KOJIMA INDUSTRIES CORPORATION

<120>  A method of producing recombinant protein

<130>  FP17-0434-00

<160>  6

<170>  PatentIn version 3.5

<210>  1
<211>  601
<212>  PRT
<213>  Artificial Sequence

<220>
<223>  SEC472

<400>  1

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

```
Gly Pro Gly Gln Tyr Gly Pro Gly Gln Gln Gly Pro Ser Ala Ser Ala
            165              170            175

Ala Ala Ala Ala Gly Ser Gly Gln Gln Gly Pro Gly Gln Tyr Gly Pro
            180              185            190

Tyr Ala Ser Ala Ala Ala Ala Ala Gly Gln Tyr Gly Ser Gly Pro Gly
            195              200            205

Gln Gln Gly Pro Tyr Gly Pro Gly Gln Ser Gly Ser Gly Gln Gln Gly
            210              215            220

Pro Gly Gln Gln Gly Pro Tyr Ala Ser Ala Ala Ala Ala Ala Gly Pro
225              230            235            240

Gly Gln Gln Gly Pro Tyr Gly Pro Gly Ser Ser Ala Ala Ala Ala Ala
            245              250            255

Gly Gln Tyr Gly Tyr Gly Pro Gly Gln Gln Gly Pro Tyr Gly Pro Gly
            260              265            270

Ala Ser Gly Gln Asn Gly Pro Gly Ser Gly Gln Tyr Gly Pro Gly Gln
            275              280            285

Gln Gly Pro Gly Gln Ser Ala Ala Ala Ala Gly Pro Gly Gln Gln
            290              295            300

Gly Pro Tyr Gly Pro Gly Ala Ser Ala Ala Ala Ala Gly Gln Tyr
305              310            315            320

Gly Pro Gly Gln Gln Gly Pro Gly Gln Tyr Gly Pro Gly Ser Ser Gly
            325              330            335

Pro Gly Gln Gln Gly Pro Tyr Gly Pro Gly Ser Ser Ala Ala Ala Ala
            340              345            350

Ala Gly Gln Tyr Gly Pro Gly Gln Gln Gly Pro Tyr Gly Pro Gly Gln
            355              360            365

Ser Ala Ala Ala Ala Gly Gln Tyr Gln Gln Gly Pro Gly Gln Gln
            370              375            380

Gly Pro Tyr Gly Pro Gly Ala Ser Gly Pro Gly Gln Gln Gly Pro Tyr
385              390              395            400

Gly Pro Gly Ala Ser Ala Ala Ala Ala Ala Gly Pro Gly Gln Tyr Gly
            405              410            415
```

```
Pro Gly Gln Gln Gly Pro Ser Ala Ser Ala Ala Ala Ala Gly Gln
        420             425                 430

Tyr Gly Ser Gly Pro Gly Gln Tyr Gly Pro Tyr Gly Pro Gly Gln Ser
        435             440                 445

Gly Pro Gly Ser Gly Gln Gln Gly Gln Gly Pro Tyr Gly Pro Gly Ala
    450             455                 460

Ser Ala Ala Ala Ala Ala Gly Gln Tyr Gly Pro Gly Gln Gln Gly Pro
465             470                 475                 480

Tyr Gly Pro Gly Gln Ser Ala Ala Ala Ala Ala Gly Pro Gly Ser Gly
        485                 490                 495

Gln Tyr Gly Pro Gly Ala Ser Gly Gln Asn Gly Pro Gly Ser Gly Gln
        500             505                 510

Tyr Gly Pro Gly Gln Gln Gly Pro Gly Gln Ser Ala Ala Ala Ala Ala
        515             520                 525

Gly Gln Tyr Gln Gln Gly Pro Gly Gln Gln Gly Pro Tyr Gly Pro Gly
    530             535                 540

Ala Ser Ala Ala Ala Ala Ala Gly Gln Tyr Gly Ser Gly Pro Gly Gln
545             550                 555                 560

Gln Gly Pro Tyr Gly Pro Gly Gln Ser Gly Ser Gly Gln Gln Gly Pro
        565             570                 575

Gly Gln Gln Gly Pro Tyr Ala Ser Ala Ala Ala Ala Ala Gly Pro Gly
        580             585                 590

Ser Gly Gln Gln Gly Pro Gly Ala Ser
        595             600


<210>  2
<211>  252
<212>  PRT
<213>  Artificial Sequence

<220>
<223>  Collagen-type4-Kai

<400>  2

Met His His His His His His Ser Ser Gly Ser Ser Lys Asp Gly Val
1                   5                   10                  15
```

```
Pro Gly Phe Pro Gly Ser Glu Gly Val Lys Gly Asn Arg Gly Phe Pro
            20              25                  30

Gly Leu Met Gly Glu Asp Gly Ile Lys Gly Gln Lys Gly Asp Ile Gly
            35              40                  45

Pro Pro Gly Phe Arg Gly Pro Thr Glu Tyr Tyr Asp Thr Tyr Gln Glu
    50              55                  60

Lys Gly Asp Glu Gly Thr Pro Gly Pro Pro Gly Pro Arg Gly Ala Arg
65                  70                  75                  80

Gly Pro Gln Gly Pro Ser Gly Pro Pro Gly Val Pro Gly Ser Pro Gly
                85                  90                  95

Ser Ser Arg Pro Gly Leu Arg Gly Ala Pro Gly Trp Pro Gly Leu Lys
            100                 105                 110

Gly Ser Lys Gly Glu Arg Gly Arg Pro Gly Lys Asp Ala Met Gly Thr
            115                 120                 125

Pro Gly Ser Pro Gly Cys Ala Gly Ser Pro Gly Leu Pro Gly Ser Pro
    130                 135                 140

Gly Pro Pro Gly Pro Pro Gly Asp Ile Val Phe Arg Lys Gly Pro Pro
145                 150                 155                 160

Gly Asp His Gly Leu Pro Gly Tyr Leu Gly Ser Pro Gly Ile Pro Gly
                165                 170                 175

Val Asp Gly Pro Lys Gly Glu Pro Gly Leu Leu Cys Thr Gln Cys Pro
            180                 185                 190

Tyr Ile Pro Gly Pro Pro Gly Leu Pro Gly Leu Pro Gly Leu His Gly
            195                 200                 205

Val Lys Gly Ile Pro Gly Arg Gln Gly Ala Ala Gly Leu Lys Gly Ser
    210                 215                 220

Pro Gly Ser Pro Gly Asn Thr Gly Leu Pro Gly Phe Pro Gly Phe Pro
225                 230                 235                 240

Gly Ala Gln Gly Asp Pro Gly Leu Lys Gly Glu Lys
                245                 250
```

```
<210>   3
<211>   315
```

<213> Artificial Sequence

<220>
<223> Resilin-Kai

<400> 3

Met His His His His His Ser Ser Gly Ser Ser Pro Glu Pro Pro
1               5               10              15

Val Asn Ser Tyr Leu Pro Pro Ser Asp Ser Tyr Gly Ala Pro Gly Gln
            20              25              30

Ser Gly Pro Gly Gly Arg Pro Ser Asp Ser Tyr Gly Ala Pro Gly Gly
        35              40              45

Gly Asn Gly Gly Arg Pro Ser Asp Ser Tyr Gly Ala Pro Gly Gln Gly
    50              55              60

Gln Gly Gln Gly Gln Gly Gln Gly Gly Tyr Ala Gly Lys Pro Ser Asp
65              70              75              80

Ser Tyr Gly Ala Pro Gly Gly Gly Asp Gly Asn Gly Gly Arg Pro Ser
            85              90              95

Ser Ser Tyr Gly Ala Pro Gly Gly Gly Asn Gly Gly Arg Pro Ser Asp
        100             105             110

Thr Tyr Gly Ala Pro Gly Gly Gly Asn Gly Gly Arg Pro Ser Asp Thr
    115             120             125

Tyr Gly Ala Pro Gly Gly Gly Gly Asn Gly Asn Gly Gly Arg Pro Ser
    130             135             140

Ser Ser Tyr Gly Ala Pro Gly Gln Gly Gln Gly Asn Gly Asn Gly Gly
145             150             155             160

Arg Pro Ser Ser Ser Tyr Gly Ala Pro Gly Gly Gly Asn Gly Gly Arg
            165             170             175

Pro Ser Asp Thr Tyr Gly Ala Pro Gly Gly Gly Asn Gly Gly Arg Pro
        180             185             190

Ser Asp Thr Tyr Gly Ala Pro Gly Gly Gly Asn Asn Gly Gly Arg Pro
        195             200             205

Ser Ser Ser Tyr Gly Ala Pro Gly Gly Gly Asn Gly Gly Arg Pro Ser
    210             215             220

19

```
Asp Thr Tyr Gly Ala Pro Gly Gly Gly Asn Gly Asn Gly Ser Gly Gly
225             230             235             240

Arg Pro Ser Ser Ser Tyr Gly Ala Pro Gly Gln Gly Gln Gly Gly Phe
            245             250             255

Gly Gly Arg Pro Ser Asp Ser Tyr Gly Ala Pro Gly Gln Asn Gln Lys
            260             265             270

Pro Ser Asp Ser Tyr Gly Ala Pro Gly Ser Gly Asn Gly Asn Gly Gly
            275             280             285

Arg Pro Ser Ser Ser Tyr Gly Ala Pro Gly Ser Gly Pro Gly Gly Arg
            290             295             300

Pro Ser Asp Ser Tyr Gly Pro Pro Ala Ser Gly
305             310             315


<210>   4
<211>   282
<212>   PRT
<213>   Artificial Sequence

<220>
<223>   elastin short

<400>   4

Met His His His His His His Ser Ser Gly Ser Ser Leu Gly Val Ser
1               5               10              15

Ala Gly Ala Val Val Pro Gln Pro Gly Ala Gly Val Lys Pro Gly Lys
            20              25              30

Val Pro Gly Val Gly Leu Pro Gly Val Tyr Pro Gly Gly Val Leu Pro
            35              40              45

Gly Ala Arg Phe Pro Gly Val Gly Val Leu Pro Gly Val Pro Thr Gly
            50              55              60

Ala Gly Val Lys Pro Lys Ala Pro Gly Val Gly Gly Ala Phe Ala Gly
65              70              75              80

Ile Pro Gly Val Gly Pro Phe Gly Gly Pro Gln Pro Gly Val Pro Leu
            85              90              95

Gly Tyr Pro Ile Lys Ala Pro Lys Leu Pro Gly Gly Tyr Gly Leu Pro
            100             105             110
```

```
                    115                      120                      125

        Gly Ala Ala Gly Lys Ala Gly Tyr Pro Thr Gly Thr Gly Val Gly Pro
            130                  135                  140

        Gln Ala Ala Ala Ala Ala Ala Ala Lys Ala Ala Ala Lys Phe Gly Ala
        145                  150                  155                  160

        Gly Ala Ala Gly Val Leu Pro Gly Val Gly Gly Ala Gly Val Pro Gly
                        165                  170                  175

        Val Pro Gly Ala Ile Pro Gly Ile Gly Gly Ile Ala Gly Val Gly Thr
                    180                  185                  190

        Pro Ala Ala Ala Ala Ala Ala Ala Ala Ala Lys Ala Ala Lys Tyr
                195                  200                  205

        Gly Ala Ala Ala Gly Leu Val Pro Gly Gly Pro Gly Phe Gly Pro Gly
            210                  215                  220

        Val Val Gly Val Pro Gly Ala Gly Val Pro Gly Val Gly Val Pro Gly
        225                  230                  235                  240

        Ala Gly Ile Pro Val Val Pro Gly Ala Gly Ile Pro Gly Ala Ala Val
                        245                  250                  255

        Pro Gly Val Val Ser Pro Glu Ala Ala Ala Lys Ala Ala Ala Lys Ala
                    260                  265                  270

        Ala Lys Tyr Gly Ala Arg Pro Gly Val Gly
                275                  280
```

```
<210>  5
<211>  468
<212>  PRT
<213>  Artificial Sequence

<220>
<223>  type I keratin 26

<400>  5

Met Ser Phe Arg Leu Ser Gly Val Ser Arg Arg Leu Cys Ser Gln Ala
1               5                   10                  15

Gly Thr Gly Arg Leu Thr Gly Gly Arg Thr Gly Phe Arg Ala Gly Asn
            20                  25                  30

Val Cys Ser Gly Leu Gly Ala Gly Ser Ser Phe Ser Gly Pro Leu Gly
```

```
Ser Val Ser Ser Lys Gly Ser Phe Ser His Gly Gly Gly Gly Leu Gly
    50              55                  60

Ser Gly Val Cys Thr Gly Phe Leu Glu Asn Glu His Gly Leu Leu Pro
65              70                  75                      80

Gly Asn Glu Lys Val Thr Leu Gln Asn Leu Asn Asp Arg Leu Ala Ser
                85                  90                  95

Tyr Leu Asp His Val Cys Thr Leu Glu Glu Ala Asn Ala Asp Leu Glu
            100                 105                 110

Gln Lys Ile Lys Gly Trp Tyr Glu Lys Tyr Gly Pro Gly Ser Gly Arg
            115                 120                 125

Gln Leu Ala His Asp Tyr Ser Lys Tyr Phe Ser Val Thr Glu Asp Leu
    130                 135                 140

Lys Arg Gln Ile Ile Ser Val Thr Thr Cys Asn Ala Ser Ile Val Leu
145                 150                 155                 160

Gln Asn Glu Asn Ala Arg Leu Thr Ala Asp Asp Phe Arg Leu Lys Cys
                165                 170                 175

Glu Asn Glu Leu Ala Leu His Gln Ser Val Glu Ala Asp Ile Asn Gly
            180                 185                 190

Leu His Arg Val Met Asp Glu Leu Thr Leu Cys Thr Ser Asp Leu Glu
        195                 200                 205

Met Gln Cys Glu Ala Leu Ser Glu Glu Leu Thr Tyr Leu Lys Lys Asn
    210                 215                 220

His Gln Glu Glu Met Lys Val Met Gln Gly Ala Ala Arg Gly Asn Val
225                 230                 235                 240

Asn Val Glu Ile Asn Ala Ala Pro Gly Val Asp Leu Thr Val Leu Leu
                245                 250                 255

Asn Asn Met Arg Ala Glu Tyr Glu Asp Leu Ala Glu Gln Asn His Glu
            260                 265                 270

Asp Ala Glu Ala Trp Phe Ser Glu Lys Ser Thr Ser Leu His Gln Gln
            275                 280                 285
```

                290                     295                        300

Glu Leu Lys Arg Asn Leu Gln Thr Leu Glu Ile Glu Leu Gln Ser Leu
305                 310                 315                 320

Leu Ala Met Lys His Ser Tyr Glu Cys Ser Leu Ala Glu Thr Glu Ser
                325                 330                 335

Asn Tyr Cys His Gln Leu Gln Gln Ile Gln Glu Gln Ile Gly Ala Met
            340                 345                 350

Glu Asp Gln Leu Gln Gln Ile Arg Met Glu Thr Glu Gly Gln Lys Leu
            355                 360                 365

Glu His Glu Arg Leu Leu Asp Val Lys Ile Phe Leu Glu Lys Glu Ile
    370                 375                 380

Glu Met Tyr Cys Lys Leu Ile Asp Gly Glu Gly Arg Lys Ser Lys Ser
385                 390                 395                 400

Thr Cys Tyr Lys Ser Glu Gly Arg Gly Pro Lys Asn Ser Glu Asn Gln
                405                 410                 415

Val Lys Asp Ser Lys Glu Glu Ala Val Val Lys Thr Val Val Gly Glu
            420                 425                 430

Leu Asp Gln Leu Gly Ser Val Leu Ser Leu Arg Val His Ser Val Glu
            435                 440                 445

Glu Lys Ser Ser Lys Ile Ser Asn Ile Thr Met Glu Gln Arg Leu Pro
    450                 455                 460

Ser Lys Val Pro
465


<210>  6
<211>  12
<212>  PRT
<213>  Artificial Sequence

<220>
<223>  His Tag

<400>  6

Met His His His His His His Ser Ser Gly Ser Ser
1               5                   10

## Description

### Technical Field

The present invention relates to a method for production of a recombinant protein on an industrial scale using microorganisms.

### Background Art

Production of recombinant proteins on an industrial scale is carried out using methods such as fed batch culture, semi-continuous culture and continuous culture.

Fed batch culture is also known as semi-batch culture. Batch culture is a culturing method in which all of the substrates necessary for culturing (the nutrients, medium components, etc.) are added to the culture medium at the start of culturing. Fed batch culture, on the other hand, is a culturing method in which the culturing is initiated in culture medium prepared with a suitable substrate concentration, and supplemental culturing is then carried out with sequential addition of each of the consumed substrates. Batch culturing and fed batch culturing are both culturing methods in which the culture solution is not removed from the culturing tank (bioreactor) until culturing is complete. For culturing systems where growth is inhibited in the presence of a high substrate concentration, fed batch culture can avoid growth inhibition by regulating the substrate concentration to an appropriately low concentration. Fed batch culture often makes it possible to achieve high cell density, allowing production of recombinant proteins at high concentration. Known methods for adding feed substrates include a method of sequential addition of the feed substrate in separate portions, a constant flow addition method in which the feed substrate is added at a constant flow rate (constant speed feeding), and an exponential feed method in which the flow rate of the feed substrate is exponentially increased.

Continuous culture is a method in which feeding of fresh medium and discharge of culture solution are carried out continuously while maintaining a constant culture solution volume, so that culturing is in a steady state without time-related change in the medium composition of the culture solution. The cell density in continuous culturing is general low compared to the cell density in fed batch culturing. Continuous culture, however, allows continuous production of recombinant proteins.

Semi-continuous culture is a method in which, after having obtained a specific volume or biomass by batch culture or fed batch culture, a portion of the culture solution including the recombinant protein is removed from the bioreactor while fresh culture medium is added to the bioreactor to continue production of the recombinant protein, and the process is repeated (Patent Literature 1).

For structural proteins such as resilin and elastin that have very high elasticity and repulsion elasticity while also having rubber-like properties, keratin, as one of the major proteins in cashmere and wool, collagen which serves to provide dynamic strength to various connective tissues, silkworm silk which is light, strong and has characteristic gloss, and spider silk which has excellent strength and ductility, methods are being devised to produce such structural proteins as recombinant proteins on an industrial scale, but many issues currently remain in regard to their implementation. Even in the case of spider silk proteins, which are being avidly researched, the production levels are low and expression of natural spider silk proteins in bacterial hosts is considered to be inefficient (Non Patent Literature 1 and Patent Literature 2).

### Citation List

### Patent Literature

[Patent Literature 1] Japanese Patent Public Inspection No. 2010-527239
[Patent Literature 2] International Patent Application Publication No. WO2015/042164

### Non Patent Literature

[Non Patent Literature 1] Appl Microbiol Biotechnol., 1998, 49(1), pp.31-38.

## Summary of Invention

### Problems to be Solved by the Invention

It is an object of the present invention to provide a method for efficiently and stably producing recombinant proteins on an industrial scale.

### Means for Solving the Problems

During the course of researching methods for producing recombinant proteins on an industrial scale by continuous culturing with transformed microorganisms, the present inventors have found that by not reusing the microorganisms whose expression has been already induced, it is possible to efficiently and stably achieve high production of desired recombinant proteins, and the invention has been completed upon this finding.

Specifically, the invention relates to the following respective inventions.

[1] A method for production of a recombinant protein using recombinant cells that express the recombinant protein under the control of an inducible promoter,

the method including continuous culturing with addition of fresh culture medium to a portion of the culture solution in which the recombinant cells have been grown,

wherein the recombinant cells whose expression of the recombinant protein has been induced are not reused.

[2] The method for production of a recombinant protein according to [1], wherein growth of the recombinant cells and induction of expression of the recombinant protein are carried out in different culturing tanks.

[3] The method for production of a recombinant protein according to [1] or [2], wherein growth of the recombinant cells is carried out by fed batch culture.

[4] A method for production of a recombinant protein using recombinant cells that express the recombinant protein under the control of an inducible promoter,

the method including repeating the following steps (A) to (E).

(A) A step of growing the recombinant cells in a culturing tank by batch culture or fed batch culture.

(B) A step of transferring a portion of the culture solution in the culturing tank to a receiving culturing tank, after the growth in step (A).

(C) A step of adding fresh culture medium to either of the two culturing tanks after the transfer in step (B), and advancing to step (A).

(D) A step of inducing expression of the recombinant protein in the other culturing tank that has not advanced to step (A) in step (C), and accumulating the recombinant protein.

(E) A step of separating and purifying the recombinant protein that has been accumulated in step (D) from the culture solution.

[5] The method for production of a recombinant protein according to any one of [1] to [4], wherein induction of expression of the recombinant protein is initiated when growth of the recombinant cells has reached the metaphase of the logarithmic growth stage.

[6] The method for production of a recombinant protein according to any one of [1] to [5], wherein induction of expression of the recombinant protein is carried out by adding an expression inducing agent to the culture solution or by varying the temperature of the culture solution.

[7] The method for production of a recombinant protein according to [4], wherein the amount of culture solution transferred to the receiving culturing tank in step (B) is 80 to 99 vol% based on the total amount of the culture solution.

[8] The method for production of a recombinant protein according to any one of [1] to [7], wherein the recombinant cells are cells in which a gene coding for the recombinant protein has been chromosomally integrated.

[9] The method for production of a recombinant protein according to any one of [1] to [8], wherein the recombinant protein is a structural protein.

[10] The method for production of a recombinant protein according to [9], wherein the structural protein is a protein derived from a protein selected from the group consisting of keratin,collagen, elastin, resilin, silkworm silk and spider silk.

[11] The method for production of a recombinant protein according to any one of [1] to [10], wherein the inducible promoter is an IPTG-inducible promoter selected from among T7 promoter, *tac* promoter, *trc* promoter, *lac* promoter and lacUV5 promoter, or a temperature-inducible promoter selected from among PR promoter and PL promoter.

**Effects of the Invention**

According to the invention it is possible to efficiently produce recombinant proteins on an industrial scale.

In expression-induced production of a recombinant protein by continuous culturing using a plasmid-introduced plasmid-type expressing strain, the issue of low productivity has been faced when using prior art technology, due to plasmid shedding, structural destabilization and reduced expression levels. According to the invention, an effect is further exhibited which allows efficient production of a recombinant protein in a stable manner without plasmid shedding, even with continuous culturing of a plasmid-type expressing strain, on a level at least comparable to a chromosomally integrated expressing strain that has the target protein integrated into its chromosomes.

**Brief Description of Drawings**

Fig. 1 is a diagram schematically showing a culturing system to be used in the method for production of a recombinant protein according to an embodiment of the invention.

Fig. 2 is a graph showing production volume of recombinant modified fibroin by repeated fed batch culture in Example 1.

Fig. 3 is a graph showing production volume of recombinant modified fibroin by repeated fed batch culture in Comparative Example 1.

Fig. 4 is a diagram schematically showing a culturing system to be used in the method for production of a recombinant protein according to the prior art.

**Embodiments for Carrying Out the Invention**

Embodiments for carrying out the invention will now be explained in further detail. However, the present invention is not limited to the embodiments described below.

[Method for production of recombinant protein]

The method for production of a recombinant protein according to this embodiment uses recombinant cells that express a recombinant protein, and it includes continuous culturing with addition of fresh culture medium to a portion of the culture solution in which the recombinant cell have been grown, without reusing the recombinant cells in which expression of the recombinant protein has been induced. The recombinant cells to be used in the production method of this embodiment are preferably recombinant cells expressing a recombinant protein under the control of an inducible promoter.

(Recombinant protein)

The recombinant protein to be produced by the production method of this embodiment (hereunder also referred to as "target protein") may be any protein that is desired to be produced on an industrial scale, and for example, it may be an industrially useful protein, or a medically useful protein, or a structural protein. Specific examples of industrially useful or medically useful proteins include enzymes, regulatory proteins, receptors, peptide hormones, cytokines, membrane or transport proteins, antigens used for vaccination, vaccines, antigen-binding proteins, immunostimulatory proteins, allergens, and full length antibodies or antibody fragments or their derivatives. Specific examples of structural proteins include spider silk, silkworm silk, keratin, collagen, elastin and resilin, as well as proteins derived from them.

Examples of spider silk- or silkworm silk-like proteins which are fibroin-like proteins include proteins including a domain sequence represented by formula 1: $[(A)_n \text{ motif-REP}]_m$ (where $(A)_n$ motif represents an amino acid sequence composed of 4 to 20 amino acid residues, and the number of alanine residues with respect to the total amino acid residues in the $(A)_n$ motif is 80% or greater; REP represents an amino acid sequence composed of 10 to 200 amino acid residues; m represents an integer of 8 to 300; multiple $(A)_n$ motifs may be identical amino acid sequences or different amino acid sequences; and multiple REP sequences may be identical amino acid sequences or different amino acid sequences). Specifically, these may be proteins including the amino acid sequence listed as SEQ ID NO: 1.

Examples of collagen-derived proteins include proteins including a domain sequence represented by formula 2: $[\text{REP2}]_o$ (where o represents an integer of 5 to 300; REP2 represents an amino acid sequence comprising Gly-X-Y, with X and Y representing any amino acid residues other than Gly; and multiple REP2 sequences may be identical amino acid sequences or different amino acid sequences). Specifically, these may be proteins including the amino acid sequence listed as SEQ ID NO: 2. The amino acid sequence listed as SEQ ID NO: 2 has the amino acid sequence listed as SEQ ID NO: 6 (tag sequence and hinge sequence) added to the N-terminus of the amino acid sequence from the 301st residue to the 540th residue corresponding to the repeat portion and motif of the partial sequence of human collagen type 4 acquired from the NCBI database (NCBI Genbank Accession No.: CAA56335.1, GI:3702452).

Examples of resilin-derived proteins include proteins including a domain sequence represented by formula 3: [REP3]$_p$ (where p represents an integer of 4 to 300; REP3 represents an amino acid sequence comprising Ser-J-J-Tyr-Gly-U-Pro; J represents any amino acid residue, and most preferably an amino acid residue selected from the group consisting of Asp, Ser and Thr; U represents any amino acid residue, and most preferably an amino acid residue selected from the group consisting of Pro, Ala, Thr and Ser; and multiple REP3 sequences may be identical amino acid sequences or different amino acid sequences). Specifically, these may be proteins including the amino acid sequence listed as SEQ ID NO: 3. The amino acid sequence listed as SEQ ID NO: 3 has the amino acid sequence listed as SEQ ID NO: 6 (tag sequence and hinge sequence) added to the N-terminus of the amino acid sequence from the 19th residue to the 321st residue of the amino acid sequence of resilin (NCBI Genbank Accession No. NP 611157, G1:24654243) wherein the 87th residue Thr is replaced by Ser, and the 95th residue Asn is replaced by Asp.

Examples of elastin-derived proteins include proteins having the amino acid sequences of NCBI Genbank Accession No. AAC98395 (human), 147076 (sheep) and NP786966 (cow). Specifically, these may be proteins including the amino acid sequence listed as SEQ ID NO: 4. The amino acid sequence listed as SEQ ID NO: 4 has the amino acid sequence listed as SEQ ID NO: 6 (tag sequence and hinge sequence) added to the N-terminus of the amino acid sequence from the 121st residue to the 390th residue of the amino acid sequence of NCBI Genbank Accession No. AAC98395.

Examples of keratin-derived proteins include *Capra hircus* type I keratin. Specifically, these may be proteins including the amino acid sequence listed as SEQ ID NO: 5 (the amino acid sequence of NCBI Genbank Accession No. ACY30466).

(Recombinant cells expressing recombinant protein)

The recombinant cells of this embodiment can be obtained, for example, by transforming a host with an expression vector having a nucleic acid sequence coding for the target protein and one or more regulatory sequences linked to the nucleic acid sequence in a functional manner.

The regulatory sequence is a sequence that regulates expression of the recombinant protein in the host (for example, a promoter, enhancer, ribosome binding sequence or transcription termination sequence), and it may be selected as appropriate depending on the type of host. The type of expression vector may be appropriately selected depending on the type of host, such as a plasmid vector, viral vector, cosmid vector, phasmid vector or artificial chromosome vector.

The host used may be any prokaryote, or any eukaryote such as yeast, filamentous fungi, insect cells, animal cells or plant cells. Preferred examples of prokaryotes include *E. coli, Bacillus subtilis, Pseudomonas, Corynebacterium* and *Lactococcus,* with *E. coli* cells being more preferred.

The expression vector used may be any one capable of autoreplication in the host cells, or capable of integrating into the host chromosomes, and containing an inducible promoter at a location that allows transcription of nucleic acid coding for the target protein.

The inducible promoter may be an inducible promoter that functions in the host cells and is capable of inducing expression of the target protein. An inducible promoter is a promoter that can regulate transcription based on the presence of an inducer (expression inducing agent), the absence of a repressor molecule, or by physical factors such as increase or reduction in temperature, osmotic pressure or pH value.

Specific examples of inducible promoters for prokaryotes as the host include T7 promoter, *tac* and *trc* promoter and *lac* and *lac*UV5 promoter, which are induced by lactose or its analog IPTG (isopropylthiol-β-D-galactoside); *araBAD* promoter which is induced by arabinose; *trp* promoter which is induced by β-indoleacrylic acid addition or tryptophan starvation and inhibited by tryptophan addition; *rhaBAD* promoter which is induced by rhamnose; *xylF* promoter and *xylA* promoter which are induced by xylose; *araBAD* promoter which is induced by arabinose; λ phage PR promoter and PL promoter which are induced by temperature increase; *phoA* promoter which is induced by phosphate starvation; and *cstA* promoter and *cstA-lacZ* promoter which is induced by glucose starvation.

The prokaryote host such as a bacterium may be a microorganism belonging to *Escherichia, Brevibacillus, Serratia, Bacillus, Microbacterium, Brevibacterium, Corynebacterium* or *Pseudomonas.*

Examples of microorganisms belonging to *Escherichia* include *Escherichia coli* BL21 (Novagen), *Escherichia coli* BL21 (DE3) (Life Technologies Corp.), *Escherichia coli* BLR (DE3) (Merck, Ltd.-Millipore), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No.49, *Escherichia coli* Rosetta (DE3) (Novagen), *Escherichia coli* TB1, *Escherichia coli* Tuner (Novagen), *Escherichia coli* Tuner (DE3) (Novagen), *Escherichia coli* W1485, *Escherichia coli* W3110 (ATCC 27325), *Escherichia coli* XL1-Blue and *Escherichia*

*coli* XL2-Blue.

Compounds of microorganisms belonging to *Brevibacillus* include *Brevibacillus agri, Brevibacillus borstenesis, Brevibacillus centrosporus, Brevibacillus formosus, Brevibacillus invocatus, Brevibacillus laterosporus, Brevibacillus limnophilus, Brevibacillus parabrevis, Brevibacillus reuszeri, Brevibacillus thermoruber, Brevibacillus brevis* 47 (FERM BP-1223), *Brevibacillus brevis* 47K (FERM BP-2308), *Brevibacillus brevis* 47-5 (FERM BP-1664), *Brevibacillus brevis* 47-5Q (JCM8975), *Brevibacillus choshinensis* HPD31 (FERM BP-1087), *Brevibacillus choshinensis* HPD31-S (FERM BP-6623), *Brevibacillus choshinensis* HPD31-OK (FERM BP-4573) and *Brevibacillus choshinensis* SP3 (Takara).

Examples of microorganisms belonging to *Serratia* include *Serratia liquefacience* ATCC14460, *Serratia entomophila, Serratia ficaria, Serratia fonticola, Serratia grimesii, Serratia proteamaculans, Serratia odorifera, Serratia plymuthica* and *Serratia rubidaea.*

Examples of microorganisms belonging to *Bacillus* include *Bacillus subtilis* and *Bacillus amyloliquefaciens.*

Examples of microorganisms belonging to *Microbacterium* include *Microbacterium ammoniaphilum* ATCC15354.

Examples of microorganisms belonging to *Brevibacterium* include *Brevibacterium divaricatum* (*Corynebacterium glutamicum*) ATCC14020, *Brevibacterium flavum* (*Corynebacterium glutamicum* ATCC14067) ATCC13826 and ATCC14067, *Brevibacterium immariophilum* ATCC14068, *Brevibacterium lactofermentum* (*Corynebacterium glutamicum* ATCC13869)ATCC13665 and ATCC13869, *Brevibacterium roseum* ATCC13825, *Brevibacterium saccharolyticum* ATCC14066, *Brevibacterium thiogenitalis* ATCC19240, *Brevibacterium album* ATCC15111 and *Brevibacterium cerinum* ATCC15112.

Examples of microorganisms belonging to *Corynebacterium* include *Corynebacterium ammoniagenes* ATCC6871 and ATCC6872, *Corynebacterium glutamicum* ATCC13032, *Corynebacterium glutamicum* ATCC14067, *Corynebacterium acetoacidophilum* ATCC13870, *Corynebacterium acetoglutamicum* ATCC15806, *Corynebacterium alkanolyticum* ATCC21511, *Corynebacterium callunae* ATCC15991, *Corynebacterium glutamicum* ATCC13020, ATCC13032 and ATCC13060, *Corynebacterium lilium* ATCC15990, *Corynebacterium melasecola* ATCC17965, *Corynebacterium thermoaminogenes* AJ12340 (FERMBP-1539) and *Corynebacterium herculis* ATCC13868.

Examples of microorganisms belonging to *Pseudomonas* include *Pseudomonas putida, Pseudomonas fluorescence, Pseudomonas brassicacearum, Pseudomonas fulva* and *Pseudomonas sp.* D-0110.

The method of introducing the expression vector into the host cells may be any method for introducing DNA into the host cells. For example, a method using calcium ion [Proc. Natl. Acad. Sci. USA, 69, 2110(1972)], a protoplast method (Japanese Unexamined Patent Application Publication SHO No. 63-248394) or the methods described in Gene, 17, 107(1982) or Molecular & General Genetics, 168, 111(1979) may be used.

Transformation of a microorganism belonging to *Brevibacillus* may be carried out by the method of Takahashi et al. (J. Bacteriol., 1983, 156:1130-1134), the method of Takagi et al. (Agric. Biol. Chem., 1989, 53:3099-3100) or the method of Okamoto et al. (Biosci. Biotechnol. Biochem., 1997, 61:202-203), for example.

Examples for the vector for introduction of the nucleic acid coding for the target protein (hereunder referred to simply as "vector") include pBTrp2, pBTac1 and pBTac2 (all commercially available from Boehringer Mannheim), pKK233-2 (Pharmacia), pSE280 (Invitrogen), pGEMEX-1 (Promega), pQE-8 (QIAGEN), pKYP10 (Japanese Unexamined Patent Application Publication SHO No. 58-110600), pKYP200 [Agric. Biol. Chem., 48, 669(1984)], pLSA1 [Agric. Biol. Chem., 53, 277(1989)], pGEL1 [Proc. Natl. Acad. Sci. USA, 82, 4306(1985)], pBluescript II SK(-) (Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM B-400), Japanese Unexamined Patent Application Publication SHO No. 60-221091], pGKA2 [prepared from *Escherichia coli* IGKA2 (FERM BP-6798), Japanese Unexamined Patent Application Publication SHO No. 60-221091], pTerm2 (US4686191, US4939094, US5160735), pSupex, pUB110, pTP5, pC194, pEG400 [J. Bacteriol., 172, 2392(1990)], pGEX (Pharmacia) and pET system (Novagen).

When *Escherichia coli* is used as the host, pUC18, pBluescriptII, pSupex, pET22b or pCold may be mentioned as suitable vectors.

Specific examples of suitable vectors for microorganisms belonging to *Brevibacillus* include pUB110, which is publicly known as a *Bacillus subtilis* vector, or pHY500 (Japanese Unexamined Patent Application Publication HEI No. 2-31682), pNY700 (Japanese Unexamined Patent Application Publication HEI No. 4-278091), pHY4831 (J. Bacteriol., 1987, 1239-1245), pNU200 (Udaka, S, Journal of Japan Society for Bioscience, Biotechnology and Agrochemistry 1987, 61:669-676), pNU100 (Appl. Microbiol. Biotechnol., 1989, 30:75-80), pNU211 (J. Biochem., 1992, 112:488-491), pNU211R2 L5 (Japanese Unexamined Patent Application Publication HEI No. 7-170984), pNH301 (Appl. Environ. Microbiol., 1992, 58:525-531), pNH326, pNH400 (J. Bacteriol., 1995, 177:745-749), pHT210 (Japanese Unexamined Patent Application Publication HEI No. 6-133782), pHT110R2L5 (Appl. Microbiol. Biotechnol., 1994, 42:358-363), or pNCO2, which is a shuttle vector between *E. coli* and a microorganism belonging to *Brevibacillus* (Japanese Unexamined Patent Application Publication No. 2002-238569).

Examples of eukaryote hosts include yeast and filamentous fungi (molds).

Examples of yeasts include yeast belonging to *Saccharomyces, Schizosaccharomyces, Kluyveromyces, Trichosporon, Schwanniomyces, Pichia, Candida, Yarrowia* and *Hansenula.* More specifically, they include *Saccharomyces cerevisiae, Schizosaccharomyces pombe, Kluyveromyces lactis, Kluyveromyces marxianus, Trichosporon pullulans, Schwanniomyces alluvius, Schwanniomyces occidentalis, Candida utilis, Pichia pastoris, Pichia angusta, Pichia methanolica, Pichia polymorpha, Pichia stipitis, Yarrowia lipolytica* and *Hansenula polymorpha.*

When yeast is used as the host cells, the expression vector preferably includes a replication origin (when amplification in the host is necessary), and a selection marker for proliferation of the vector in *E. coli*, an inducible promoter and terminator for expression of the recombinant protein in the yeast, and a selection marker for the yeast.

When the expression vector is a nonintegrated vector, it preferably also includes an autonomously replicating sequence (ARS). This can increase the stability of the expression vector in the cells (Myers, A.M., et al.(1986) Gene 45:299-310).

Examples of vectors when yeast are used as the host include YEP13 (ATCC37115), YEp24 (ATCC37051), YCp50 (ATCC37419), YIp, pHS19, pHS15, pA0804, pHIL3O1, pHIL-S1, pPIC9K, pPICZα, pGAPZα and pPICZ B.

Specific examples of inducible promoters, when yeast are used as the host, include galactose inducible gal 1 promoter and gal 10 promoter; copper inducible CUP 1 promoter; thiamine inducible *nmt1* promoter; and methanol inducible AOX1 promoter, AOX2 promoter, DHAS promoter, DAS promoter, FDH promoter, FMDH promoter, MOX promoter and ZZA1, PEX5-, PEX8- and PEX14-promoters.

The method of introducing the expression vector into the yeast may be any method for introducing DNA into yeast, and examples include electroporation methods (Methods Enzymol., 194, 182(1990)), spheroplast methods (Proc. Natl. Acad. Sci.,USA, 81, 4889(1984)), lithium acetate methods (J. Bacteriol., 153, 163(1983)), and the method described in Proc. Natl. Acad. Sci. USA, 75, 1929(1978).

Examples of filamentous fungi include fungi belonging to *Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus* and *Rhizomucor.*

Specific examples of filamentous fungi include *Acremonium alabamense, Acremonium cellulolyticus, Aspergillus aculeatus, Aspergillus awamori, Aspergillus oryzae, Aspergillus sake, Aspergillus sojae, Aspergillus tubigensis, Aspergillus niger, Aspergillus nidulans, Aspergillus parasiticus, Aspergillus ficuum, Aspergillus phoeicus, Aspergillus foetidus, Aspergillus flavus, Aspergillus fumigatus, Aspergillus japonicus, Trichoderma viride, Trichoderma harzianum Trichoderma reseei, Chrysosporium lucknowense, Thermoascus, Sporotrichum, Sporotrichum cellulophilum, Talaromyces, Thielavia terrestris, Thielavia, Neurospora crassa, Fusarium oxysporus, Fusarium graminearum, Fusarium venenatum, Humicola insolens, Penicillium chrysogenum, Penicillium camemberti, Penicillium canescens, Penicillium emersonii, Penicillium funiculosum, Penicillium griseoroseum, Penicillium purpurogenum, Penicillium roqueforti, Myceliophtaora thermophilum, Mucor ambiguus, Mucor circinelloides, Mucor fragilis, Mucor hiemalis, Mucor inaequisporus, Mucor oblongiellipticus, Mucor racemosus, Mucor recurvus, Mocor saturninus, Mocor subtilissmus, Ogataea polymorpha, Phanerochaete chrysosporium, Rhizomucor miehei, Rhizomucor pusillus* and *Rhizopus arrhizus.*

Specific examples of inducible promoters when filamentous fungi are used as the host include salicylic acid inducible PRIa promoter; cycloheximide inducible Placc promoter; and quinic acid inducible Pqa-2 promoter.

Introduction of the expression vector into filamentous fungi may be carried out using a method known in the prior art. Such methods include the method of Cohen et al. (calcium chloride method) [Proc. Natl. Acad. Sci. USA, 69:2110(1972)], the protoplast method [Mol. Gen. Genet., 168:111(1979)], the competent method [J. Mol. Biol., 56:209(1971)] and electroporation.

The recombinant cells of this embodiment may have nucleic acid coding for the target protein integrated into the chromosomes (chromosomal DNA). The nucleic acid coding for the target protein is linked in a functional manner to one or more regulatory sequences. The regulatory sequences in this case may be exogenous or endogenous.

The method of integrating the nucleic acid coding for the target protein into the host chromosomes may be any publicly known method, and for example, it may be a λred method implementing a recombinant mechanism for double chain break repair of λ phage, a Red/ET homologous recombination method, or a transfer method using transposon activity with pUT-mini Tn5. For example, a "pUTmini-Tn5 Kit transposon gene transfer kit" by Biomedal may be used to integrate nucleic acid coding for the target protein into host chromosomes, following the method described in the kit manual.

(Culturing method)

The production method of this embodiment includes continuous culturing with addition of fresh culture medium to a portion of the culture solution in which the recombinant cells have been grown, without reusing the recombinant cells in which expression of the recombinant protein has been induced. Here, "without reusing" means that the recombinant cells in which expression of the recombinant protein has been induced are not used in culturing for subsequent regrowth. The culturing may be carried out under aerobic conditions, such as deep aeration stirring culture.

The culturing method for this embodiment may be, for example, a method of growing recombinant cells by batch culture or fed batch culture, and then dividing the culture solution containing the grown recombinant cells into cells for inducing expression of the recombinant protein and cells for culturing for subsequent regrowth, and adding fresh culture medium to the culture solution for culturing for subsequent regrowth, and repeating the culturing procedure. The amount of culture solution for inducing expression of the recombinant protein may be 70 to 99 vol%, preferably 80 to 99 vol% and more preferably 90 to 99 vol%, for example, based on the total culture solution. The amount of culture solution divided out for culturing for subsequent regrowth may be 1 to 30 vol%, preferably 1 to 20 vol% and more preferably 1 to 10 vol%, for example, based on the total culture solution.

When the culturing for growth is to be fed batch culture, the feed substrate solution may include one or more nutrients of the medium component, for example. Feeding of the feed substrate solution may be carried out as a continuous system or a discontinuous system, according to a method known in the technical field. The feed amount is not particularly restricted, and feeding may be carried out by combining a linear constant coefficient system, a linear increment system, a stepwise increment system or an exponential feed system, with the proliferated cell count as the index. The amount of cells can be confirmed based on the dry cell weight, wet cell weight or colony formation units. Feeding allows the recombinant cells to be cultured to a high density.

The method of culturing according to another embodiment may be, for example, a method of continuously feeding fresh culture medium and discharging the culture solution, while maintaining approximately constant culture solution volume, and conducting culturing in a steady state such that the medium composition in the culture solution does not change with time (continuous culture), with the discharged culture solution being used as culture solution for inducing expression of the recombinant protein.

The type of culture medium used for culturing is not particularly restricted. Any natural culture medium or synthetic culture medium may be used, so long as it contains a carbon source, nitrogen source and inorganic salts that can be assimilated by the recombinant cells, and allows efficient culturing of the recombinant cells.

The carbon source may be any one that can be assimilated by the recombinant cells, including carbohydrates such as glucose, fructose, sucrose and molasses containing them, starch and starch hydrolysate, organic acids such as acetic acid and propionic acid, and alcohols such as ethanol and propanol.

Examples of nitrogen sources include ammonia, ammonium salts of inorganic acids or organic acids such as ammonium chloride, ammonium sulfate, ammonium acetate and ammonium phosphate, and other nitrogen-containing compounds, as well as peptone, meat extract, yeast extract, corn steep liquor, casein hydrolysate, soybean meal and soybean meal hydrolysate, and various fermentative microbes and their digestion products.

Examples of inorganic salts that may be used include monopotassium phosphate, dipotassium phosphate, magnesium phosphate, magnesium sulfate, sodium chloride, ferrous sulfate, manganese sulfate, copper sulfate and calcium carbonate.

The culturing temperature is 15 to 40°C, for example. The pH of the culture solution during culturing is preferably kept at 3.0 to 9.0. The pH of the culture solution can be adjusted using an inorganic acid, organic acid, alkali solution, urea, calcium carbonate, ammonia or the like.

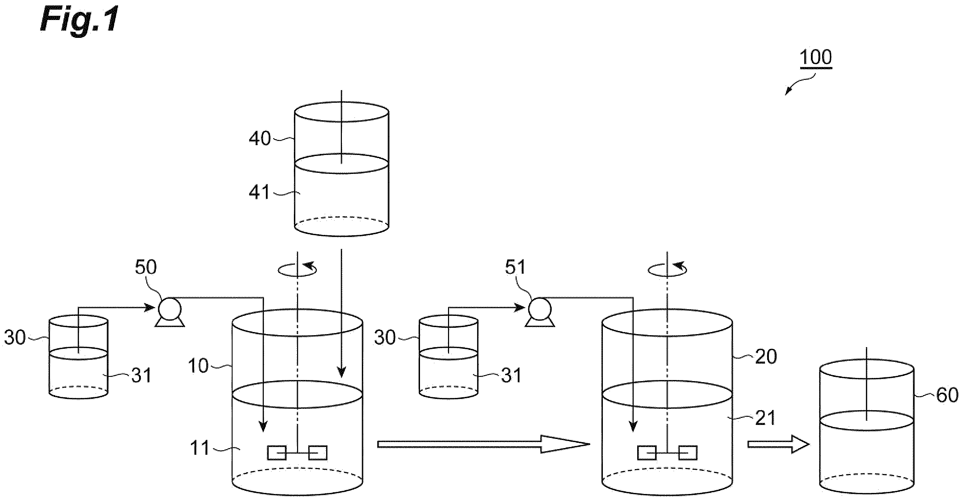

A production method according to the first embodiment will now be described in detail with reference to Fig. 1. Fig. 1 is a diagram schematically showing a culturing system to be used in the method for production of a recombinant protein according to an embodiment of the invention. The culturing system 100 shown in Fig. 1 comprises two tanks, a culturing tank 10 and a culturing tank 20. The culturing tank 10 is a culturing tank for growth of recombinant cells. A feed substrate solution 31 is supplied to the culturing tank 10 from a feed substrate solution storage tank 30 connected through a pump 50.

Upon reaching the anaphase from the metaphase during the logarithmic growth stage, the recombinant cells grown in the culturing tank 10 are transferred to the culturing tank 20 as culture solution 11. The amount of transferred culture solution 11 may be 70 to 99 vol%, preferably 80 to 99 vol% and more preferably 90 to 99 vol%, for example, based on the total amount of the culture solution 11. After transfer, a portion of the culture solution 11 remains in the culturing tank 10, and fresh culture medium 41 is supplied from a culture medium storage tank 40, after which growth of the recombinant cells is repeated.

The culturing tank 20 is a culturing tank for expression of a recombinant protein. In the culturing tank 20, an inducible promoter is activated to induce expression of the recombinant protein. The activation of an inducible promoter referred to here means activation of transcription of a nucleic acid coding for a recombinant protein by an inducible promoter. For example, when an inducible promoter is activated by the presence of an inducing substance (expression inducing agent), addition of the inducing substance to the culturing tank 20 can induce expression of the recombinant protein. Also, when the inducible promoter is activated by increase or decrease in temperature, for example, expression of the recombinant protein can be induced by heating or cooling the culturing tank 20 to control the temperature in the culture solution 21.

Expression of the recombinant protein is induced in the culturing tank 20 for 10 to 20 hours, for example. During this time, the feed substrate solution 31 may be supplied to the culturing tank 20 from the feed substrate solution storage tank 30 connected via a pump 51. The feed substrate solution 31 includes one or more nutrients in the culture medium. Supplying the feed substrate solution 31 can increase the efficiency of inducing expression of the recombinant protein.

After conducting induction of expression of the recombinant protein for the prescribed time period, the culture solution 21 is transferred to a separation purification tank 60. The amount of transferred culture solution 21 may be 50 to 100 vol%, preferably 80 to 100 vol% and more preferably 90 to 100 vol% and even more preferably 100 vol%, for example, based on the total amount of the culture solution 21. The expressed recombinant protein is separated and purified at the separation purification tank 60.

After the culture solution 21 has been transferred, the recombinant cells that have been grown in the culturing tank 10 are transferred to the culturing tank 20, and expression of the recombinant protein is induced. Growth of the recombinant cells at the culturing tank 10 and induction of expression of the recombinant protein at the culturing tank 20 are then continuously repeated.

Since growth of the recombinant cells and induction of expression of the recombinant protein are carried out in different culturing tanks (the culturing tank 10 and culturing tank 20) in the culturing system 100, the recombinant cells in which expression of the recombinant protein has been induced are not repeatedly reused. This construction allows efficient production of the recombinant protein in a stable manner, even when the cycle is repeated. When a plasmid-type expressing strain is used as the recombinant cells, plasmid shedding is minimized as well.

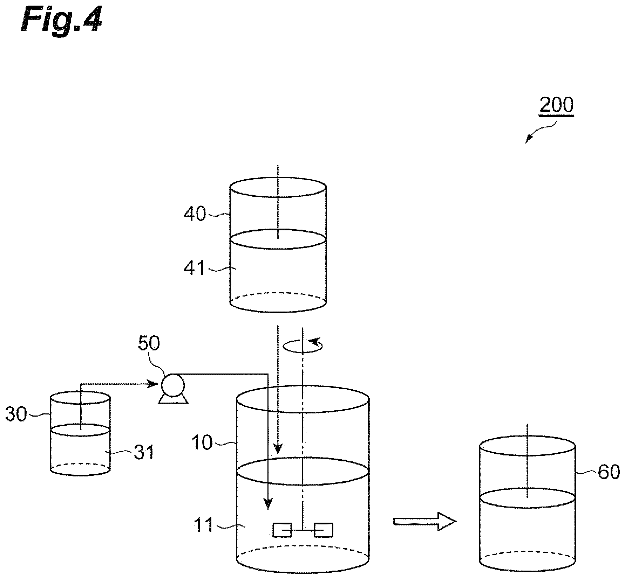

Fig. 4 is a diagram schematically showing a culturing system to be used in the method for production of a recombinant protein according to the prior art. The culturing system 200 shown in Fig. 4 comprises only a single culturing tank 10. Both growth of the recombinant cells and induction of expression of the recombinant protein are carried out in the culturing tank 10. After conducting induction of expression of the recombinant protein for the prescribed time period in the culturing tank 10, the culture solution 11 is transferred to a separation purification tank 60. After transfer, a portion of the culture solution 11 remains in the culturing tank 10, fresh culture medium 41 is supplied from a culture medium storage tank 40, and growth of the recombinant cells and induction of expression of the recombinant protein are repeated. With the construction of the culturing system 200, the production volume of the recombinant protein is markedly reduced when the cycle is repeated. Furthermore, plasmid shedding tends to occur when a plasmid-type expressing strain is used as the recombinant cells.

The production method according to one embodiment may include repetition of the following steps (A) to (E).

(A) A step of growing the recombinant cells in a culturing tank by batch culture or fed batch culture.
(B) A step of transferring a portion of the culture solution in the culturing tank to a receiving culturing tank, after the growth in step (A).
(C) A step of adding fresh culture medium to either of the two culturing tanks after the transfer in step (B), and advancing to step (A).
(D) A step of inducing expression of the recombinant protein in the other culturing tank that has not advanced to step (A) in step (C), and accumulating the recombinant protein.
(E) A step of separating and purifying the recombinant protein that has been accumulated in step (D) from the culture solution.

When step (D) is to be carried out in a receiving culturing tank, the amount of culture solution transferred to the receiving culturing tank in step (B) may be 70 to 99 vol%, preferably 80 to 99 vol% and more preferably 90 to 99 vol%, based on the total amount of the culture solution. When step (C) is to be carried out in a receiving culturing tank, the amount of culture solution transferred to the receiving culturing tank in step (B) may be 1 to 30 vol%, preferably 1 to 20 vol% and more preferably 1 to 10 vol%, based on the total amount of the culture solution.

(Inducing expression of the recombinant protein)

Induction of expression of the recombinant protein is carried out by activating transcription by an inducible promoter (transcription of nucleic acid coding for the target protein). Activation of an inducible promoter may be carried out by a method known in the technical field, depending on the type of inducible promoter.

For example, when using an inducible promoter that is activated by the presence of an inducing substance (expression inducing agent), addition of the inducing substance to the culture solution can induce expression of the recombinant protein. The inducing substance may be added to the culture solution all at once or in several portions, or it may be added to the culture solution as a continuous feed. Feeding may also be by addition of the inducing substance to the feed substrate solution. The amount of inducing substance added may be set according to the type of inducing substance and inducible promoter, but as an example it may be in the range of 0.1 to 30 μg and preferably in the range of 0.5 to 20 μg, for 1 g of dry weight of the recombinant cells.

When the inducible promoter is to be activated by increase or decrease in temperature, for example, expression of the recombinant protein may be induced by increasing or decreasing the temperature of the culture solution. For example, when using λ phage PR promoter or PL promoter which is activated by temperature increase, expression of the recombinant protein during growth may be suppressed when the temperature of the culture solution during growth is in the range of 20 to 37°C, and expression of the recombinant protein can then be induced by increasing the culture solution temperature to 38 to 44°C. In order to lessen the effect of heat shock protein during the process, the pH of the culture solution during growth may be adjusted to 6.5 to 7.5, as described in Japanese Unexamined Patent Application Publication HEI No. 6-292563, and the pH of the culture solution varied to 4.5 to 6.5 at the start of inducing expression of the recombinant protein, thereby allowing more stable induction of expression.

There is no particular restriction on the period from the stage of growth of the recombinant cells until the stage of inducing expression of the recombinant protein, and it may be appropriately set according to the culturing system configuration and the production process design. From the viewpoint of efficient production of the recombinant protein, it is preferred to initiate induction of expression of the recombinant protein when growth of the recombinant cells has reached the metaphase to the anaphase of the logarithmic growth stage.

Growth of the recombinant cells begins from the lag phase or induction phase (the period of delayed increase in the initial cell count), through the logarithmic growth stage (the period of logarithmic increase to twice the cell count per unit time), and reaches the stationary phase (the period where no net change is seen in the number of cells). The metaphase of the logarithmic growth stage is the period in which the cell count is midway between the cell count in the lag phase and the cell count in the stationary phase, and the anaphase of the logarithmic growth stage is the period from the metaphase until the stationary phase. As a specific example of the period for initiating induction of expression of the recombinant protein, for recombinant cells wherein the $OD_{600}$ value at the stationary phase is approximately 150, it is preferably the period in which the $OD_{600}$ value has reached 30 to 110, more preferably the period in which it has reached 40 to 90, and even more preferably the period in which it has reached 50 to 80.

The time for inducing expression of the recombinant protein may be a time length until the predetermined production volume has been obtained, which will depend on the type of host used and the target protein. Since the production rate varies depending on the culturing conditions such as the temperature of the culture solution, it is not necessary to absolutely specify the time for inducing expression of the recombinant protein. The time for inducing expression of the recombinant protein may also be set to match progression to separation and purification of the recombinant protein in the subsequent step. For industrial production, it is preferred to set the time for induction of expression of the recombinant protein so as not to affect growth of the recombinant cells being carried out in parallel, or transfer of the grown recombinant cells.

The culture solution in which expression of the recombinant protein has been induced is used for the following separation and purification of the target recombinant protein.

(Separation and purification of recombinant protein)

Separation and purification of the recombinant protein may be carried out by a commonly used method. For example, if the recombinant protein is to be expressed in the cells in dissolved form, then after the culturing for inducing expression of the recombinant protein is completed, the host cells (recombinant cells) may be collected by centrifugal separation and suspended in an aqueous buffer, and then the host cells may be disrupted using an ultrasonic disruptor, French press, Manton Gaulin homogenizer or Dyno-Mill, to obtain a cell-free extract. The cell-free extract is centrifuged to obtain a supernatant, from which a purified preparation of the recombinant protein may be obtained using one or a combination of methods commonly used for isolation and purification of proteins, such as a solvent extraction method, a salting out method with ammonium sulfate or the like, a desalting method, a precipitation method with an organic solvent, an anion exchange chromatography method using a resin such as diethylaminoethyl (DEAE)-Sepharose or DIAION HPA-75 (product of Mitsubishi Chemical Corp.), a cation exchange chromatography method using a resin such as S-Sepharose FF (product of Pharmacia), a hydrophobic chromatography method using a resin such as Butyl Sepharose or Phenyl Sepharose, a gel filtration method using a molecular sieve, an affinity chromatography method, a chromatofocusing method, or an electrophoresis method such as isoelectric focusing.

When the recombinant protein is expressed in an insoluble form in the cells, the recombinant protein is collected in insoluble form as a precipitated fraction, by collecting and then disrupting and centrifuging the host cells in the same manner. The insoluble recombinant protein that is recovered may be solubilized using a protein denaturing agent. After the procedure, isolation and purification may be carried out in the same manner as described above to obtain a purified preparation of the recombinant protein.

When the recombinant protein has been secreted extracellularly, the recombinant protein may be recovered from the culture supernatant. That is, by treating the culture solution using a method such as centrifugal separation, it is possible to obtain the culture supernatant, from which a purified preparation of the recombinant protein may then be obtained by isolation and purification in the same manner as described above.

**Examples**

The present invention will now be explained in greater detail based on examples. However, the present invention is not limited to the examples described below.

[Example 1]

[(1) Preparation of modified fibroin-expressing strain]

(Preparation of plasmid-type expressing strain)

Modified fibroin (hereunder referred to as "SEC472") having the amino acid sequence listed as SEQ ID NO: 1 was designed based on the nucleotide sequence and amino acid sequence for fibroin from *Nephila clavipes* (GenBank Accession No.: P46804.1, GI:1174415).

The amino acid sequence listed as SEQ ID NO: 1 is the amino acid sequence, having substitutions, insertions and deletions of amino acid residues as compared to *Nephila clavipes* fibroin, for increased productivity, and further having the amino acid sequence represented by SEQ ID NO: 6 (tag sequence and hinge sequence) added to the N-terminus.

Nucleic acid coding for SEC472 was then synthesized. The nucleic acid had an NdeI site added at the 5'-end and an EcoRI site added downstream from the stop codon. The nucleic acid was cloned in a cloning vector (pUC118). The nucleic acid was then subjected to restriction enzyme treatment with NdeI and EcoRI for cleavage, after which it was recombined with the protein expression vector pET-22b(+) to obtain an expression vector.

*E. coli* BLR(DE3) was transformed with expression vector pET-22b(+), to obtain a plasmid-introduced plasmid-type expressing strain SEC659.

(Preparation of chromosomally integrated expressing strain)

A chromosomally integrated expressing strain was prepared using pUTmini-Tn5 Kit by Biomedal.

Nucleic acid coding for modified fibroin (SEC472) having the amino acid sequence listed as SEQ ID NO: 1 was synthesized. The nucleic acid had a NotI site added at the 5'-end and downstream from the stop codon. A plasmid was constructed having this nucleic acid inserted at the NotI site of pUTmini-Tn5 Km. Next, a strain obtained by transforming S17-1 λpir with this plasmid was mixed with *E. coli* BLR(DE3) at a 1:1 ratio, and cultured in LB- and Km-containing plate culture medium. A chromosomally integrated expressing strain SEC714 integrating the nucleic acid in its chromosomes was obtained from the strains exhibiting Km resistance and Ap sensitivity.

[(2) Seed culturing]

The plasmid-type expressing strain SEC659 and chromosomally integrated expressing strain SEC714 prepared in (1) were each cultured for 15 hours in 2 mL of LB medium containing ampicillin. The same culture solution was added to 100 mL of seed culture medium containing ampicillin (Table 1) to an $OD_{600}$ of 0.005, and flask culturing was conducted to an $OD_{600}$ of 5 (approximately 15 hours) while keeping the culture solution temperature at 30°C, to obtain seed culture solutions.

[Table 1]

| Seed culture medium | |
| --- | --- |
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

[(3) Preparation of main culture medium]

The composition of the main culture medium is shown in Table 2.

[Table 2]

| Main culture medium | |
|---|---|
| Reagent | Concentration (g/L) |
| Glucose | 10.0 |
| $KH_2PO_4$ | 22.0 |
| $FeSO_4 \cdot 7H_2O$ | 0.04 |
| $CaCl_2 \cdot 2H_2O$ | 0.04 |
| $MgSO_4 \cdot 7H_2O$ | 2.4 |
| Yeast Extract | 11.25 |
| Defoaming agent (Adeka, LG-295S) | 1.0 (mL/L) |

A 500 mL portion of main culture medium (Table 2) was added to a culturing tank, and sterilization treatment was carried out for 20 minutes in an autoclave (TOMY LSX-500) at 121°C. After cooling to 37°C, 28% to 30% ammonia water (01266-88 by Kanto Kagaku Co., Ltd.) was used to adjust the pH to 6.1 to 6.3.

[(4) Preparation of feed substrate solution]

The composition of the feed substrate solution is shown in Table 3.

[Table 3]

| Feed substrate solution | |
|---|---|
| Reagent | Concentration (g/L) |
| Glucose | 600.0 |
| $MgSO_4 \cdot 7H_2O$ | 10.0 |

A prescribed amount of feed substrate solution was added to the feeding pot, and sterilization treatment was carried out for 20 minutes in an autoclave (TOMY LSX-500) at 121°C.

[(5) Repeated fed batch culture and expression induction]

Recombinant modified fibroin was produced with plasmid-type expressing strain SEC659 and chromosomally integrated expressing strain SEC714, using the culturing system 100 illustrated in Fig. 1. A TSC-A1L-5 culturing tank (product of Takasugi Seisakusho, 1 L capacity) was used for the culturing tank 10 and the culturing tank 20. The recombinant modified fibroin was produced by repeated fed batch culture in which culture solution (~95%) fed up to the prescribed cell density was used for expression induction, while fresh culture medium was added to the remainder of the culture solution (~5%) and fed batch culture was repeated.

The main culture medium (0.5 L initial medium volume) was loaded into the culturing tank 10, and the obtained seed culture solution was added to an $OD_{600}$ of 0.05. Main culturing was conducted with the temperature of the culture solution kept at 37°C, and using 30% ammonia water and a 4 M phosphoric acid solution (Wako Pure Chemical Industries, Ltd.) for constant control to pH 6.9. Aerated stirring was carried out so that the dissolved oxygen concentration in the culture solution was maintained at 30-40% dissolved oxygen saturated concentration. A massflow controller (MPC0005BBRN0100D0 by Azbil Corp.) was used for control.

For the main culturing, feeding of the feed substrate solution 31 from the feed substrate solution storage tank 30 into the culturing tank 10 was initiated at the point where the dissolved oxygen saturated concentration exceeded about 55% after having fallen below about 30%. The feed rate of the feed substrate solution 31 was constant rate feeding at 6 g/hr.

After continuing fed batch culture in the culturing tank 10 until the $OD_{600}$ of the culture solution 11 reached about 60, approximately 95% of the culture solution 11 was transferred aseptically to the culturing tank 20 through a sampling line (not shown). After transfer, IPTG (expression inducing agent) was added to the culturing tank 20 to 0.2 mM, and expression induction of the recombinant modified fibroin was initiated.

After initiating expression induction, culturing was continued in the culturing tank 10 under the same conditions as the main culture, except that the feed substrate solution 31 was fed from the feed substrate solution storage tank 30 to the culturing tank 20 at a feed rate of 9 g/hr. After expression induction in the culturing tank 20 for approximately 16 hours, the total amount of culture solution 21 was transferred to the separation purification tank 60 and used for separation and purification of recombinant modified fibroin.

Fresh culture medium 41 was added from the culture medium storage tank 40 to the culturing tank 10 after transfer of the culture solution 11 to the culturing tank 20 (with approximately 5% of the culture solution 11 remaining), and main culturing and fed batch culturing were carried out in the culturing tank 10 under the same conditions as described above. The culture solution 11 in the culturing tank 10 that had been cultured by fed batch culture to an $OD_{600}$ of about 60 in the same manner as described above was transferred to the culturing tank 20 after the total amount of culture solution 21 had been transferred into the separation purification tank 60, and expression induction of the recombinant modified fibroin was carried out. The repeated fed batch culturing was repeated 8 times, including the main culturing.

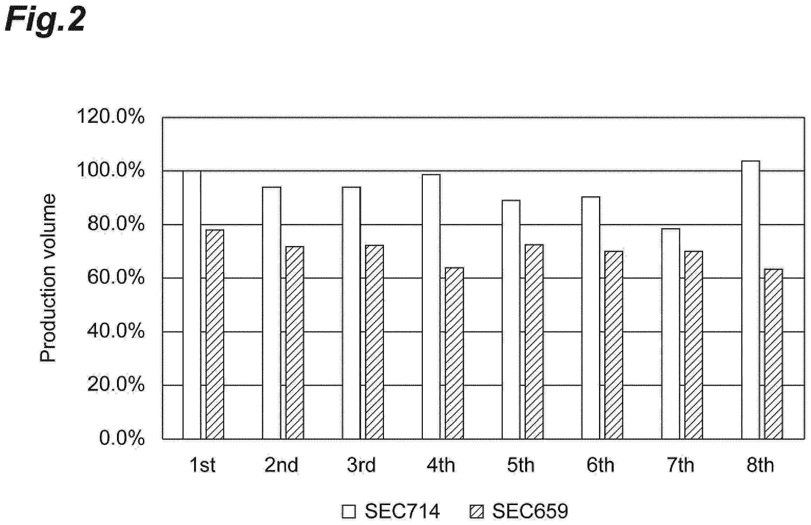

Fig. 2 shows the results of analyzing the production volume of recombinant modified fibroin in the 8 repeated fed batch cultures with plasmid-type expressing strain SEC659 and chromosomally integrated expressing strain SEC714. The production volumes shown in Fig. 2 are represented as relative values with the production volume of recombinant modified fibroin in the first repeated fed batch culture with SEC714 as 100%.

As shown in Fig. 2, both the plasmid-type expressing strain SEC659 and chromosomally integrated expressing strain SEC714 maintained the production volume of recombinant modified fibroin in the first repeated fed batch culture even up through the 8th repeated fed batch culture.

The plasmid retention in plasmid-type expressing strain SEC659 was confirmed by the following method. The culture solution after each repeated fed batch culturing was diluted with LB medium and seeded on LB agar medium (culture medium A). After culturing for 18 to 20 hours in a thermostatic bath at 37°C, the 50 to 100 grown colonies were transferred onto ampicillin-added LB agar medium (culture medium B) using a sterilized toothpick. The strains transplanted in culture medium B were cultured for 12 to 18 hours in a thermostatic bath at 37°C, and then the number of colonies transferred from culture medium A to culture medium B and the number of colonies formed on culture medium B were counted and calculation was performed by the following formula.

$$\text{Plasmid retention} = (\text{number of colonies formed on culture}$$
$$\text{medium B})/(\text{number of colonies transferred from culture medium A to}$$
$$\text{culture medium B})$$

As a result, the plasmid-type expressing strain SEC659 maintained a plasmid retention of 100% up through addition of IPTG in the 8th repeated fed batch culture.

Incidentally, stable continuous production was also possible by the method described above for recombinant proteins derived from keratin, collagen, elastin and resilin, including the amino acid sequences listed as SEQ ID NO: 2 to 5.

[Comparative Example 1]

[Repeated fed batch culture and expression induction]

Recombinant modified fibroin was produced with plasmid-type expressing strain SEC659 and chromosomally integrated expressing strain SEC714, using a conventional culturing system 200 as illustrated in Fig. 4. A TSC-A1L-5 culturing tank (product of Takasugi Seisakusho, 1 L capacity) was used for the culturing tank 10 and culturing tank 20. The recombinant modified fibroin was produced by repeated fed batch culture in which fresh culture medium was added to a portion of the culture solution in which expression had been induced (~5%) and fed batch culture was repeated.

Repeated fed batch culturing and expression induction were carried out under essentially the same conditions as Example 1, except that expression induction of the recombinant modified fibroin was carried out continuously not in the culturing tank 20 but in the culturing tank 10 in which the main culturing and fed batch culturing had been carried out.

That is, after fed batch culture had been continued in the culturing tank 10 until the $OD_{600}$ of the culture solution 11 reached about 60, IPTG (expression inducing agent) was subsequently added to the culturing tank 10 to 0.2 mM, and expression induction of the recombinant modified fibroin was initiated. After initiating expression induction, the feed substrate solution 31 was fed from the feed substrate solution storage tank 30 to the culturing tank 10 at a feed rate of 9 g/hr. After expression induction in the culturing tank 10 for approximately 16 hours, approximately 95% of the culture solution 11 was transferred to the separation purification tank 60 and used for separation and purification of recombinant modified fibroin. After the transfer, fresh culture medium 41 was added from the culture medium storage tank 40 to the culturing tank 10 (in which approximately 5% of the culture solution 11 remained), and main culturing, fed batch culturing and expression induction of recombinant modified fibroin were carried out under the same conditions. The repeated fed batch culturing was repeated 5 times.

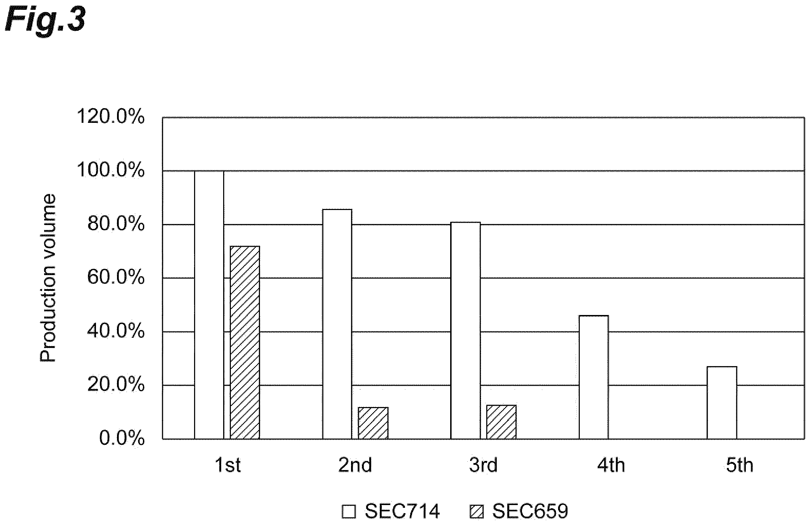

Fig. 3 shows the results of analyzing the production volume of recombinant modified fibroin in the 5 repeated fed batch cultures with plasmid-type expressing strain SEC659 and chromosomally integrated expressing strain SEC714. The production volumes shown in Fig. 3 are represented as relative values with the production volume of recombinant modified fibroin in the first repeated fed batch culture with SEC714 as 100%.

As shown in Fig. 3, when repeated fed batch culture was repeated only in the culturing tank 10 (that is, when the recombinant cells in which recombinant modified fibroin expression had been induced were reused), the production volume of recombinant modified fibroin after the 2nd culture was extremely reduced, with plasmid-type expressing strain SEC659. The plasmid retention measured in the same manner as Example 1 was 0%, at the point of addition of IPTG in the second repeated fed batch culture. With chromosomally integrated expressing strain SEC714, on the other hand, the production volume of recombinant modified fibroin was reduced with each repeated fed batch culture, although a more stable production volume was exhibited than with plasmid-type expressing strain SEC659.

**Reference Signs List**

10, 20: Culturing tank, 11, 21: culture solution, 30: feed substrate solution storage tank, 31: feed substrate solution, 40: culture medium storage tank, 41: culture medium, 50, 51: pump, 60: separation purification tank, 100, 200: culturing system.

**Claims**

1. A method for production of a recombinant protein using recombinant cells that express the recombinant protein under the control of an inducible promoter, the method comprising continuous culturing with addition of fresh culture medium to a portion of the culture solution in which the recombinant cells have been grown, wherein the recombinant cells whose expression of the recombinant protein has been induced are not reused.

2. The method for production of a recombinant protein according to claim 1, wherein growth of the recombinant cells and induction of expression of the recombinant protein are carried out in different culturing tanks.

3. The method for production of a recombinant protein according to claim 1 or 2, wherein growth of the recombinant cells is carried out by fed batch culture.

4. A method for production of a recombinant protein using recombinant cells that express the recombinant protein under the control of an inducible promoter, the method comprising repeating the following steps (A) to (E),

    (A) a step of growing the recombinant cells in a culturing tank by batch culture or fed batch culture,
    (B) a step of transferring a portion of the culture solution in the culturing tank to a receiving culturing tank, after the growth in step (A),
    (C) a step of adding fresh culture medium to either of the two culturing tanks after the transfer in step (B), and advancing to step (A),
    (D) a step of inducing expression of the recombinant protein in the other culturing tank that has not advanced to step (A) in step (C), and accumulating the recombinant protein, and
    (E) a step of separating and purifying the recombinant protein that has been accumulated in step (D) from the culture solution.

5. The method for production of a recombinant protein according to any one of claims 1 to 4, wherein induction of expression of the recombinant protein is initiated when growth of the recombinant cells has reached the metaphase of the logarithmic growth stage.

6. The method for production of a recombinant protein according to any one of claims 1 to 5, wherein induction of expression of the recombinant protein is carried out by adding an expression inducing agent to the culture solution or by varying the temperature of the culture solution.

7. The method for production of a recombinant protein according to claim 4, wherein the amount of culture solution transferred to the receiving culturing tank in step (B) is 80 to 99 vol% based on the total amount of the culture solution.

8. The method for production of a recombinant protein according to any one of claims 1 to 7, wherein the recombinant cells are cells in which a gene coding for the recombinant protein has been chromosomally integrated.

9. The method for production of a recombinant protein according to any one of claims 1 to 8, wherein the recombinant protein is a structural protein.

10. The method for production of a recombinant protein according to claim 9, wherein the structural protein is a protein derived from a protein selected from the group consisting of keratin, collagen, elastin, resilin, silkworm silk and spider silk.

11. The method for production of a recombinant protein according to any one of claims 1 to 10, wherein the inducible promoter is an IPTG-inducible promoter selected from among T7 promoter, tac promoter, trc promoter, lac promoter and lacUV5 promoter, or a temperature-inducible promoter selected from among PR promoter and PL promoter.

Fig.1

Fig.2

# *Fig.3*

# Fig.4

## INTERNATIONAL SEARCH REPORT

International application No.

PCT/JP2017/027958

**A. CLASSIFICATION OF SUBJECT MATTER**
*C12P21/02*(2006.01)i, *C12N1/00*(2006.01)i

According to International Patent Classification (IPC) or to both national classification and IPC

**B. FIELDS SEARCHED**

Minimum documentation searched (classification system followed by classification symbols)
C12P21/02, C12N1/00

Documentation searched other than minimum documentation to the extent that such documents are included in the fields searched
Jitsuyo Shinan Koho        1922-1996   Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2015/137441 A1  (Tokyo Institute of Technology), 17 September 2015 (17.09.2015), examples (Family: none) | 1-3,5-6,8-11 |
| A | JP 2010-527239 A  (Boehringer Ingelheim RCV GmbH & Co. KG.), 12 August 2010 (12.08.2010), entire text & US 2011/0045531 A1     & WO 2008/142028 A1 & EP 2160469 A1          & KR 10-2010-0017315 A | 1-11 |

☐ Further documents are listed in the continuation of Box C.      ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered    to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 September 2017 (11.09.17) | 26 September 2017 (26.09.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010527239 A
- WO 2015042164 A
- JP 63248394 A
- JP 58110600 A
- JP 60221091 A
- US 4686191 A
- US 4939094 A
- US 5160735 A
- JP 2031682 A
- JP 4278091 A
- JP 7170984 A
- JP 6133782 A
- JP 2002238569 A
- JP 6292563 A

**Non-patent literature cited in the description**

- *Appl Microbiol Biotechnol.,* 1998, vol. 49 (1), 31-38
- *Proc. Natl. Acad. Sci. USA,* 1972, vol. 69, 2110
- *Gene,* 1982, vol. 17, 107
- *Molecular & General Genetics,* 1979, vol. 168, 111
- **TAKAHASHI et al.** *J. Bacteriol.,* 1983, vol. 156, 1130-1134
- **TAKAGI et al.** *Agric. Biol. Chem.,* 1989, vol. 53, 3099-3100
- **OKAMOTO et al.** *Biosci. Biotechnol. Biochem.,* 1997, vol. 61, 202-203
- *Agric. Biol. Chem.,* 1984, vol. 48, 669
- *Agric. Biol. Chem.,* 1989, vol. 53, 277
- *J. Bacteriol.,* 1990, vol. 172, 2392
- *J. Bacteriol.,* 1987, 1239-1245
- **UDAKA, S.** *Journal of Japan Society for Bioscience, Biotechnology and Agrochemistry,* 1987, vol. 61, 669-676
- *Appl. Microbiol. Biotechnol.,* 1989, vol. 30, 75-80
- *J. Biochem.,* 1992, vol. 112, 488-491
- *Appl. Environ. Microbiol.,* 1992, vol. 58, 525-531
- *J. Bacteriol.,* 1995, vol. 177, 745-749
- *Appl. Microbiol. Biotechnol.,* 1994, vol. 42, 358-363
- **MYERS, A.M. et al.** *Gene,* 1986, vol. 45, 299-310
- *Methods Enzymol.,* 1990, vol. 194, 182
- *Proc. Natl. Acad. Sci.,USA,* 1984, vol. 81, 4889
- *J. Bacteriol.,* 1983, vol. 153, 163
- *Proc. Natl. Acad. Sci. USA,* 1978, vol. 75, 1929
- *Mol. Gen. Genet.,* 1979, vol. 168, 111
- *J. Mol. Biol.,* 1971, vol. 56, 209